(19) **Europäisches Patentamt**

**European Patent Office**

**Office européen des brevets**

(11) Veröffentlichungsnummer: **0 433 741 A1**

(12) ## EUROPÄISCHE PATENTANMELDUNG

(21) Anmeldenummer: 90123054.0

(22) Anmeldetag: 01.12.90

(51) Int. Cl.⁵: **G01N 27/18**

(30) Priorität: 04.12.89 DE 3940034

(43) Veröffentlichungstag der Anmeldung:
26.06.91 Patentblatt 91/26

(84) Benannte Vertragsstaaten:
**DE FR GB IT NL**

(71) Anmelder: **ROSEMOUNT GMBH & CO.**
**Wilhelm-Rohn-Strasse 51**
W-6450 Hanau 1(DE)

(72) Erfinder: **Krause, Hans, Dr.**
**Eichendorffstrasse 4**
**W-6350 Bad Nauheim(DE)**

(74) Vertreter: **Haar, Lucas Heinz Jörn, Dipl.-Ing.**
**Königsberger Strasse 23**
**W-6360 Friedberg/Hessen 1(DE)**

(54) **Verfahren und Vorrichtung zur Detektion von fluidischen, insbesondere gasförmigen oder flüssigen Medien, die nach dem Prinzip der Wärmeleitfähigkeit arbeiten.**

(57) Es werden ein Verfahren und eine Vorrichtung zur Detektion von fluidischen, insbesondere gasförmigen oder flüssigen Medien, die nach dem Prinzip der Wärmeleitfähigkeit arbeiten vorgestellt. In einem Strömungsberuhigungsraum (9) ist ein Aggregat in der Position (13) angeordnet, das mit Strömungsverengungsquerschnitten und Strömungserweiterungsquerschnitten zur Vernichtung der kinetischen Energie des in den Strömungsberuhigungsraum eingeströmten Mediums versehen ist. Die Erfindung zeichnet sich dadurch aus, daß mit einem kleinen Aggregat und durch geschickte Anordnung von Bauelementen der Strömungstechnik eine Berührung von Medium und Sensorfläche (7) lediglich per Diffusion, also strömungslos, erreicht wird. Dadurch wird die Qualität des Sensorsignals erheblich verbessert.

FIG.1

EP 0 433 741 A1

## VERFAHREN UND VORRICHTUNG ZUR DETEKTION VON FLUIDISCHEN, INSBESONDERE GASFÖRMIGEN ODER FLÜSSIGEN MEDIEN, DIE NACH DEM PRINZIP DER WÄRMELEITFÄHIGKEIT ARBEITEN

Die Erfindung betrifft ein Verfahren und eine Vorrichtung zur Detektion von fluidischen, insbesondere gasförmigen oder flüssigen, strömenden Medien, die nach dem Prinzip der Wärmeleitfähigkeit arbeiten.

Der Gegenstand der Erfindung gehört gattungsmäßig zu den sogenannten "Wärmeleitfähigkeitsanalysatoren". Ein Wärmeleitfähigkeits-Analysator dient unter anderem zur kontinuierlichen Erfassung von Gaskonzentrationen.

Ein derartiger Wärmeleitfähigkeits-Anlalysator, wie er Stand der Technik ist, ist beispielsweise im Prospekt der Leybold AG mit der Nummer 43-330.01 und dem Titel "Hydros 100" beschrieben.

Die Messzelle eines derartigen Wärmeleitfähigkeits-Analysators, wie er im oben genannten Prospekt beschrieben wird, wird beispielsweise auf maximal 50 Grad Celsius thermostatisiert. Die eingebauten Thermofühler werden mit Hilfe eines elektrischen Stromes aufgeheizt. Die Temperatur der Thermofühler und damit der elektrische Widerstand des im Thermofühler eingebauten Ohmschen Widerstands hängen nur vom Wärmetransport ab, der durch das umgebende Gas von dem Thermofühler zur kälteren Wand erfolgt.

Bei sonst konstanten Bedingungen ist der Wärmetransport proportional zur Wärmeleitfähigkeit des Gases zwischen dem Fühler und der Wand.

Durch Verschalten von vier Thermofühlern, siehe hierzu den oben genannten Prospekt, zu einer Wheatstoneschen Brücke kann als Brücken-Diagonal-Spannung ein konzentrations-proportionales Meßsignal abgegriffen werden. Eine nachgeschaltete Auswertelektronik setzt dieses Meßsignal in standardisierte Signalgrößen um und führt sie zum Anzeige-Instrument, sowie zu den Signal-Buchsen.

Weitere Einzelheiten sind dem oben genannten Prospekt zu entnehmen.

Der Erfindung liegen folgende Aufgaben zugrunde:

Die bekannten Verfahren und Vorrichtungen zur Detektion von fluidischen, insbesondere gasförmigen oder flüssigen, Medien, die nach dem Pronzip der Wärmeleitfähigkeit arbeiten, sollen grundsätzlich verbessert werden.

Das Anwendungsgebiet der Erfindung soll sich hierbei nicht alleine auf den Typ der Wärmeleitfähigkeitsanalysatoren, wie sie im Prospekt der Firma Leybold AG, Nummer 43 330.01, beschrieben werden, beschränken.

Die Aufgabenstellung der vorliegenden Erfindung bezieht sich hingegen generell auf Sensoren in der Analytik, die physikalische Größen von fluidischen Medien, insbesondere von gasförmigen oder flüssigen ein- oder mehrkomponentigen Stoffen, detektieren sollen.

Damit Änderungen der Stoffzusammensetzung und damit der physikalischen Größen schnell erfasst werden können, sind bei Verfahren und Vorrichtungen des Standes der Technik große Mengendurchsätze oder Volumenströme nötig. Dies hat zur Folge, daß die Geschwindigkeit am Sensorort, beziehungsweise Messort, groß ist.

Hierdurch ergibt sich in sehr nachteiliger Weise eine große Strömungsabhängigkeit des Meßsignals. Scherkräfte, dynamische und thermische Effekte spielen dabei eine Rolle.

Bisher wurde dieser Nachteil durch sogenannte Ringkammern zu unterdrücken versucht. Der Nachteil der Ringkammerlösung besteht in dem relativ großen Raumbedarf und in den notwendigerweise langen Kanälen der Ringkammer und der Querbohrungen, die zu den Sensoren führen.

Die Erfindung macht sich zur Aufgabe, die störenden Strömungen von vornherein zu unterdrükken. Es gehört weiterhin zur Aufgabe, bei gasförmigen Medien die Diffusion im Bereich der sensitiven Fläche der Sensoren oder Detektoren zu verbessern.

Es soll eine Unabhängigkeit von der Strömung selbst erzielt werden. Die Messung der Wärmeleitfähigkeit soll also nicht durch die Strömungssituation am Sensor verfälscht werden. Der Sensor soll gar nicht erst einer Strömung ausgesetzt sein.

Der Sensor soll auf kürzestem, das heißt schnellstem Wege per Diffusion mit dem zu sensierenden Gas in Berührung kommen. Die Ansprechzeit soll verkürzt werden.

Die Vorrichtung zur Durchführung des Wärmeleitfähigkeitsanalysenverfahrens soll kleiner und leichter als vergleichbare Vorrichtungen des Standes der Technik sein. Außerdem soll die Vorrichtung auf einfache Weise in größere Anlagen integrierbar sein.

Die gestellten Aufgaben werden erfindungsgemäß dadurch gelöst, daß zumindest ein Teil der Strömung so weit unterdrückt wird, daß die sensitive Fläche des Detektors Werte feststellt, die strömungsunabhängig sind.

In einem bevorzugten Ausführungsbeispiel wird vorgeschlagen, daß zumindest ein Teil der Strömung so weit unterdrückt wird, daß die sensitive Fläche eines Ohmschen Widerstandselements bei Berührung mit dem Medium strömungsunabhängige Widerstandsänderungen erfährt.

Eine sehr kostengünstige Vorrichtung zur Durchführung der erfindungsgemäßen Verfahren besteht darin, daß ein Strömungsberuhigungsraum vorgesehen ist, der mit dem Strömungskanal einerseits und einer Kammer, in der der Sensor angeordnet ist (Sensorkammer), in Verbindung steht.

Dabei kann vorgesehen sein, daß im Strömungsberuhigungsraum ein Aggregat mit Strömungsverengungsquerschnitten und Strömungserweiterungsquerschnitten zur Vernichtung der kinetischen Energie des in den Strömungsberuhigungsraum eingeströmten Mediums vorgesehen ist.

Ein Ausführungsbeispiel zur Vernichtung der kinetischen Energie des strömenden fluidischen Mediums besteht darin, daß im Strömungsberuhigungsraum ein Labyrinthaggregat vorgesehen ist.

Alternativ kann vorgesehen werden, daß im Strömungsberuhigungsraum ein Siebaggregat angeordnet ist.

Weiterhin wird vorgeschlagen, daß das Siebaggregat aus einem oder mehreren Sieben besteht, von denen mindestens ein Sieb als Gitter ausgebildet und vorzugsweise durch ein Ätzverfahren hergestellt worden ist.

Dabei kann vorgesehen werden, daß die Siebe rechteckige, insbesondere quadratische, oder hexagonale Öffnungen aufweisen.

Eine besonders wirkungsvolle Vernichtung der kinetischen Energie wird dadurch erreicht, daß das Siebaggregat aus mehreren koaxial und durch Drehung versetzt zueinander angeordneten Einzelsieben besteht.

In einem weiteren Ausführungsbeispiel wird vorgeschlagen, daß im Strömungsberuhigungsraum ein Paket von koaxial und durch Drehung versetzt zueinander angeordneten Lochplatten vorgesehen ist.

Durch die Erfindung werden folgende Vorteile erreicht:

Die gestellten Aufgaben werden gelöst. Die störenden Strömungen im Bereich des Sensors werden von vornherein unterdrückt. Es wird insbesondere die Diffusion im Bereich der sensitiven Fläche der Thermofühler erheblich verbessert. Die Vorrichtung zur Durchführung des Wärmeleitfähigkeitsanalysenverfahrens ist kleiner als vergleichbare Vorrichtungen des Standes der Technik. Die Ansprechzeit wird verkürzt.

Weitere Einzelheiten der Erfindung, der Aufgabenstellung und der erzielten Vorteile sind der Beschreibung mehrerer Ausführungsbeispiele der Erfindung zu entnehmen.

Diese Ausführungsbeispiele werden anhand von fünf Figuren erläutert:

Figur 1 zeigt eine Vorrichtung zur Detektion von gasförmigen, strömenden Medien.

Figur 2 zeigt ein Labyrinthaggregat, wie es in der Vorrichtung gemäß Figur 1 zur Anwendung kommen kann.

Figur 3 zeigt ein Siebaggregat, wie es in einer Vorrichtung gemäß 1 zur Anwendung kommen kann.

Die Figuren 4 und 5 zeigen ein Paktet koaxial zueinander angeordneter Lochplatten, wie es in einer Vorrichtung gemäß 1 zur Anwendung kommen kann.

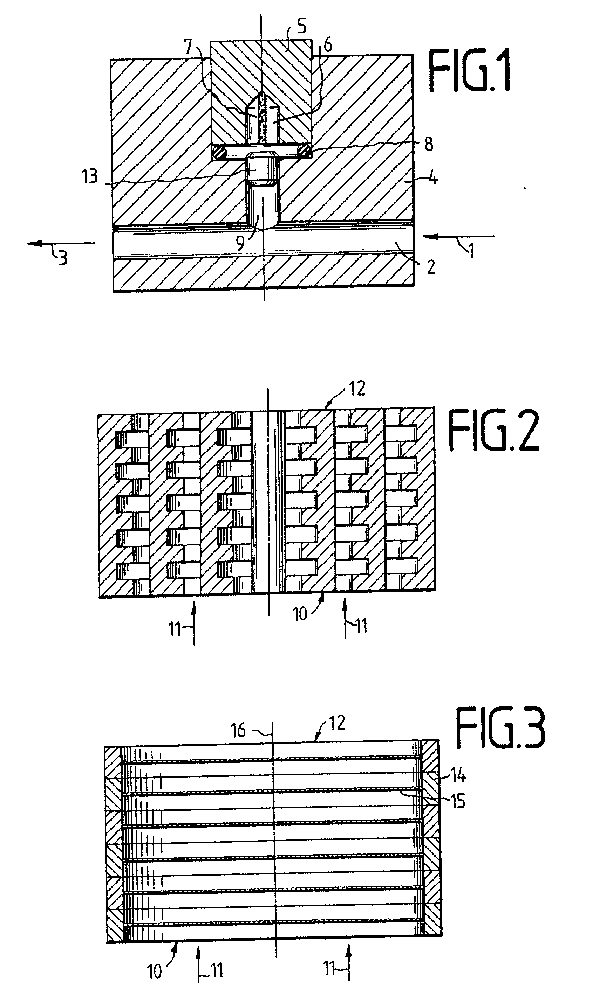

In Figur 1 ist in schematischer Form, und zwar in einer Schnittdarstellung, eine Detektionsvorrichtung für ein Gas dargestellt. Das zu detektierende Gas strömt in Richtung des Pfeils 1 in den Kanal 2. Mit dem Pfeil 3 wird das ausströmende Gas bezeichnet.

In einem Aufnahmekörper 4 befindet sich das Sensorgehäuse 5 mit einer Messkammer 6. In der Messkammer befindet sich während der Messung strömungsfreies Gas. In der Messkammer ist ein Ohmscher Widerstand untergebracht, dessen sensitive Fläche mit 7 bezeichnet ist.

Das Sensorgehäuse ist gegenüber dem Aufnahmekörper durch die Dichtung 8 abgedichtet.

9 ist ein Abzweigkanal, der als Strömungsberuhigungsraum ausgebildet ist.

Innerhalb des Strömungsberuhigungsraums können Vorrichtungen 13 vorgesehen sein, die durch besondere strömungstechnische Maßnahmen die kinetische Energie des in den Beruhigungsraum strömenden Mediums vernichten.

Diese Vernichtung wird dadurch erzielt, daß im Strömungsberuhigungsraum ein Aggregat mit Strömungsverengungsquerschnitten und Strömungserweiterungsquerschnitten, beziehungsweise mit Entspannungsräumen vorgesehen ist.

Durch die Expansionssprünge wird bei Gas die der Strömung innewohnende kinetische Energie abgebaut.

In den Figuren 2 bis 5 werden mehrere Ausführungsbeispiele für ein solches Aggregat (13) dargestellt.

So zeigt Figur 2 ein Labyrinth in der Form einer an sich bekannten Labyrinth-Dichtung. Die am Eingangsquerschnitt 10 der Strömung 11 innewohnende kinetische Energie wird über die axiale Länge des Labyrinths abgebaut, so daß am Ausgangsquerschnitt 12 des Labyrinths nach Figur 2 Strömungslosigkeit herrscht.

Bei dem Ausführungsbeispiel nach Figur 3 ist ein Aggregat, bestehend aus mehreren Sieben vorgesehen, von denen eines mit 14 bezeichnet ist. Der zum Sieb 14 gehörende Siebboden trägt die Bezugsziffer 15.

Die Siebe sind koaxial angeordnet. Durch Drehung um die Achse 16 sind die Sieböffnungen versetzt zueinander angeordnet, so daß Strömungsquerschnittsverengungen und Strömungsquerschnittserweiterungen entstehen. Das Siebaggregat nach Figur 3 wird in der Position 13, siehe Figur 1, eingebaut. Analog zum Aggregat nach Figur 2 sind in Figur 3 die Zuströmrichtung durch die Pfeile 11 bezeichnet. Auch in Figur 3 sind der Eingangsquerschnitt mit 10 und der Ausgangsquerschnitt mit 12 bezeichnet.

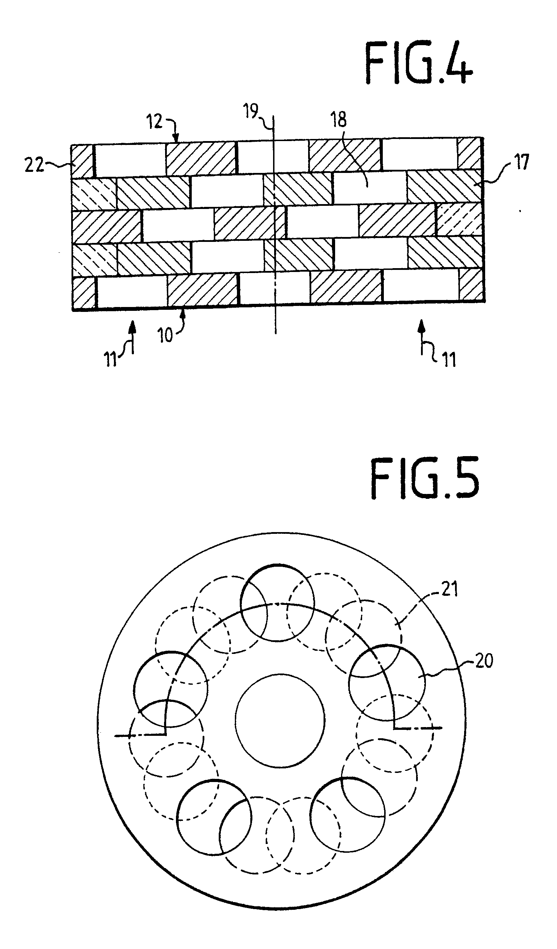

In den Figuren 4 und 5 wird ein Paket von koaxial zueinander angeordneten Lochplatten gezeigt. Eine dieser Lochplatten ist mit der Ziffer 17 bezeichnet. Diese Platte weist mehrere Löcher, beziehungsweise Ausstanzungen 18 auf.

Figur 4 ist eine schematische Schnittdarstellung, aus der ersichtlich ist, daß durch die versetzte Anordnung der Löcher Verengungsquerschnitte und Erweiterungsquerschnitte aufeinander folgen.

Die versetzte Anordnung wird dadurch erreicht, daß die einzelnen Lochplatten in Bezug auf die gemeinsame Achse 19 verdreht zueinander angeordnet sind.

Mit der Draufsicht nach Figur 5 soll schematisch gezeigt werden, daß die in einer oberen Platte 22 angebrachten Löcher, die mit ausgezogenen Kreislinien dargestellt sind und von denen eines mit 20 bezeichnet ist, um einen gewissen Winkel verdreht, das heißt versetzt, angeordnet sind in Bezug auf die Löcher einer untenliegenden Platte 17. Die Löcher der untenliegenden Platte sind gestrichelt dargestellt. Eines dieser Löcher trägt die Bezugsziffer 21.

Das Lochplattenpaket nach den Figuren 4 und 5 ist in der Position 13, siehe Figur 1, angeordnet.

Auch bei dem Ausführungsbeispiel gemäß Figur 4 wird durch die Pfeile 11 die Einströmrichtung bezeichnet. 10 ist der Eingangsquerschnitt. 12 bezeichnet den Austrittsquerschnitt.

Die Ausführungsbeispiele nach den Figuren 2, 3 und 4, beziehungsweise 5, erfüllen die eingangs beschriebene Aufgabe, die am Eingangsquerschnitt 10 auftreffende energiereiche Strömung zu beruhigen, das heißt, die kinetische Energie der Strömung zu vernichten, so daß am Ausgangsquerschnitt 12 Strömungslosigkeit herrscht mit der Folge, daß in der Messkammer die sensitive Fläche per Diffusion umspült wird.

Liste der Einzelteile

| | |
|---|---|
| 1 | Pfeil |
| 2 | Kanal |
| 3 | Pfeil |
| 4 | Aufnahmekörper |
| 5 | Sensorgehäuse |
| 6 | Kammer |
| 7 | sensitive Fläche, Sensor, Sensorfläche |
| 8 | Dichtung |
| 9 | Abzweigkanal, Strömungsberuhigungsraum |
| 10 | Eingangsquerschnitt |
| 11 | Pfeile |
| 12 | Ausgangsquerschnitt |
| 13 | Aggregat, Vorrichtung, Position |
| 14 | Sieb |
| 15 | Siebboden |
| 16 | Achse |
| 17 | Lochplatte |
| 18 | Loch, Ausstanzung |
| 19 | Achse |
| 20 | Loch |
| 21 | Loch |
| 22 | obere Platte |

**Ansprüche**

1. Verfahren zur Detektion von fluidischen, insbesondere gasförmigen oder flüssigen, strömenden Medien, das nach dem Prinzip der Wärmeleitfähigkeit arbeitet, dadurch **gekennzeichnet,** daß zumindest ein Teil der Strömung so weit unterdrückt wird, daß die sensitive Fläche des Detektors Werte feststellt, die strömungsunabhängig sind.

2. Verfahren nach Anspruch 1, das mit einem Detektor arbeitet, der ein Ohmsches Widerstandselement als Sensor umfasst, dadurch **gekennzeichnet,** daß zumindest ein Teil der Strömung so weit unterdrückt wird, daß die sensitive Fläche des Ohmschen Widerstandselements bei Berührung mit dem Medium strömungsunabhängige Widerstandsänderungen erfährt.

3. Vorrichtung zur Durchführung der Verfahren nach den Ansprüchen 1 und/oder 2 mit mindestens einem Strömungskanal, dadurch **gekennzeichnet,** daß ein Strömungsberuhigungsraum (9) vorgesehen ist, der mit dem Strömungskanal (2) einerseits und einer Kammer (6), in der der Sensor (7) angeordnet ist (Sensorkammer), in Verbindung steht.

4. Vorrichtung nach einem oder mehreren der vorangegangenen Ansprüche, dadurch **gekennzeichnet,** daß im Strömungsberuhigungsraum (9) ein Aggregat (13) mit Strömungsverengungsquerschnitten und Strömungserweiterungsquerschnitten zur Vernichtung der kinetischen Energie des in den Strömungsberuhigungsraum (9) eingeströmten Mediums vorgesehen ist.

5. Vorrichtung nach einem oder mehreren der vorangegangenen Ansprüche, dadurch **gekennzeichnet,** daß im Strömungsberuhigungsraum (9) ein Labyrinthaggregat vorgesehen ist.

6. Vorrichtung nach einem oder mehreren der vorangegangenen Ansprüche, dadurch **gekennzeichnet**, daß im Strömungsberuhigungsraum (9) ein Siebaggregat vorgesehen ist.

7. Vorrichtung nach einem oder mehreren der vorangegangenen Ansprüche, dadurch **gekennzeichnet**, daß das Siebaggregat aus einem oder mehreren Sieben (14) besteht, von denen mindestens ein Sieb als Gitter ausgebildet und vorzugsweise durch ein Ätzverfahren hergestellt worden ist.

8. Vorrichtung nach einem oder mehreren der vorangegangenen Ansprüche, dadurch gekennzeichnet, daß die Siebe (14) rechteckige, insbesondere quadratische, Öffnungen aufweisen.

9. Vorrichtung nach einem oder mehreren der vorangegangenen Ansprüche, dadurch **gekennzeichnet**, daß die Siebe (14) hexagonale Öffnungen aufweisen.

10. Vorrichtung nach einem oder mehreren der vorangegangenen Ansprüche, dadurch **gekennzeichnet**, daß das Siebaggregat aus mehreren koaxial und durch Drehung versetzt zueinander angeordneten Einzelsieben (14) besteht.

11. Vorrichtung nach einem oder mehreren der vorangegangenen Ansprüche, dadurch **gekennzeichnet**, daß im Strömungsberuhigungsraum (9) ein Paket von koaxial und durch Drehung versetzt zueinander angeordneten Lochplatten vorgesehen ist.

FIG.1

FIG.2

FIG.3

# FIG.4

# FIG.5

Europäisches
Patentamt

**EUROPÄISCHER
RECHERCHENBERICHT**

Nummer der Anmeldung

**EP 90 12 3054**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | DE-A-1 773 480   (CHARBONNAGES DE FRANCE)<br>* Seite 2, Absatz 1; Seite 4, Absatz 2; Seite 5, Absatz 5 - Seite 6, Absatz 2; Figur *<br>− − − | 1-4,6 | G 01 N<br>27/18 |
| A | | 5,7-11 | |
| A | DE-C-7 468 60   (ALLGEMEINE ELEKTRICITÄTS-GESELLSCHAFT IN BERLIN)<br>* Seite 1, Zeile 36 - Seite 2, Zeile 52; Figur 1 *<br>− − − | 1-4 | |
| A | FR-A-2 116 513   (LEYBOLD-HERAEUS-VERWALTUNG GmbH)<br>* Seite 1, Zeile 14 - Seite 2, Zeile 2; Figuren 5,6 *<br>− − − | 1-4 | |
| A | US-A-3 106 088   (KIESELBACH)<br>* Spalte 1, Zeile 1 - Spalte 2, Zeile 9; Spalte 4, Zeilen 25-49; Spalte 5, Zeilen 54-68; Figuren 3A,4 *<br>− − − | 1-4,6,7, 11 | |
| A | EP-A-0 281 966   (THE PERKIN-ELMER CORP.)<br>* Spalte 3, Zeile 24 - Spalte 5, Zeile 21; Zusammenf.; Figuren 1,6,8 *<br>− − − | 1-4,6 | |
| A | GB-A-2 131 180   (YOKOGAWA HOKUSHIN ELECTRIC CORP.)<br>* Seite 1, Zeilen 5-16,70-111; Zusammenf.; Figuren 1,2,8c *<br>− − − − − | 1 | |

| | RECHERCHIERTE SACHGEBIETE (Int. Cl.5) |
|---|---|
| | G 01 N |

Der vorliegende Recherchenbericht wurde für alle Patentansprüche erstellt

| Recherchenort | Abschlußdatum der Recherche | Prüfer |
|---|---|---|
| Den Haag | 14 März 91 | BOSMA R.A.P. |